# EUROPEAN PATENT SPECIFICATION

(11) **EP 2 373 212 B1**
(45) Date of publication and mention of the grant of the patent: **16.01.2013**
(21) Application number: 09805931.4
(22) Date of filing: 26.11.2009
(51) Int. Cl.: A61B 5/00

(54) **DEVICE**
VORRICHTUNG
DISPOSITIF

(30) Priority: 05.12.2008 GB 0822270
(43) Date of publication of application: 12.10.2011
(73) Proprietor: Accunostics Limited, Croy, Inverness IV2 5PG (GB)
(72) Inventor: DAVIES, Oliver, Croy, Inverness IV2 5PG (GB)
(74) Representative: Kinsler, Maureen Catherine
(86) International application number: PCT/GB2009/002762
(87) International publication number: WO 2010/063987

(56) References cited:
- EP-A1- 1 764 030
- DE-A1- 10 237 692
- US-A1- 2002 170 823
- US-A1- 2003 176 183

## Description

### Field of Invention

The present invention relates to a device for use with a test strip and meter system for measuring the concentration of a substance in a fluid, for example an analyte in a physiological fluid from a patient, such as glucose in a blood sample.

### Background of the Invention

Diabetes is one of the most widespread non-infectious diseases. It is estimated that around 4% of the world's population suffer from diabetes. Furthermore, the prevalence of diabetes is increasing, particularly in developed countries. Complications associated with diabetes include an increased risk of suffering a heart attack, stroke, blood circulation disorders, kidney damage, blindness and nerve conduction disorders which can result in foot or leg amputations.

Assessing the concentration of glucose in the blood is an established way of both diagnosing and managing diabetes. Diabetics, in particular insulin-dependent diabetics, are advised to monitor their blood glucose levels several times a day in order to adapt and improve treatment plans. Due to the number of times blood glucose levels should be measured, it is highly preferable that diabetics are able to self-monitor their blood glucose levels without the need for medical supervision. Because of this, self-monitoring glucose measuring devices have been developed. These are now very commonly used by diabetics to control and monitor their glucose levels.

Known self-monitoring glucose measuring devices generally comprise a handheld measurement device and a disposable test strip, with the test strip typically including one or more chemical reagents, and in many cases two or more electrodes, for example a measurement or working electrode and a reference electrode. The vast majority of systems are electrochemical, with the meter designed to receive a specific test strip in a dedicated test strip port.

Upon insertion of a test strip in to the strip port connector of a compatible meter the meter recognises the presence of the test strip and automatically turns on. The meter may then display the calibration code, the time date or other information. The meter may then prompt the user to apply a sample of blood to the test strip. Once the sample has been applied to the test strip the meter will recognise the presence of a sample on the test strip and begin the test. The meter applies a potential difference between the reference electrode and the measuring electrode(s) thereby causing a signal(s) to be generated by the measuring electrode (s) of the test strip. This signal is measured by the meter and used to calculate the concentration or amount of analyte of interest in the test sample. The meter may apply more complex controlling signals to the test strip such varying potentials and bias off periods.

Typically meters and strips are designed only to work together, and not in combination with other makes of meter or strip. However, this makes it is very hard to improve the performance of the system without the need to introduce a new meter to the market and existing manufacturers cannot easily design new and different strips with improved performance for use in existing maters. So for an existing manufacturer to Improve the performance of a glucose monitoring system, it is generally necessary to design a new strip and new meter combination. Since meters are typically given away or sold at a loss the process of providing improved performance to existing users requires the manufacturer to swap a user's old meter for a new one free of charge. This is very costly, time consuming and wasteful.

EP1764030 describes a blood sugar measuring device having an adapter allowing it to be connected to a portable device. US2003/176183 describes a glucose measurement module connected to a hand held processing device. DE10237692 describes a telemedicine system having a universal adapter, a functional module and a process module. U820021170823 describes an apparatus having a portable tester and a base operating independently from each other.

### Summary of the Invention

According to the present invention, there is provided an adapter for an analyte measuring device for measuring the amount of analyte in a test sample, typically a biological fluid, the device comprising a meter and a test strip, the adapter comprising a meter interface for connecting to a meter designed for use with a test strip of one design, and a test strip interface for connecting to a test strip of a different design, and means for adapting the at least one output from the test strip of different design for use by the test meter and/or an at least one output from the test meter for use by the test strip different design.

By providing an adapter that can be fitted between a test strip and a meter, signals can be modified to allow new or Improved test strip designs to be used with meters already in the market place. In other words this adaptor makes an alternative test strip design compatible with a meter that was designed for a specific and different test strip. Furthermore it is possible to design several different adaptors each designed to create compatibility between one single test strip design and several different target meters. By doing so one test strip design may be compatible with multiple meters all of which were designed to use other test strip designs.

The meter interface and the test strip interface may comprise one or more connectors, the meter interface connectors and the test strip interface connectors having different configurations, the adapter including means for connecting the meter interface connectors and the test strip interface connectors.

The meter interface and the test strip interface may comprise one or more connectors, the meter interface connectors and the test strip interface connectors having the substantially the same configurations, the adapter including means for connecting the meter interface connectors and the test strip interface connectors.

The connectors may be electrical connectors for passing electrical signals between the test strip and the meter.

Where there are different numbers of meter interface connectors and test strip interface connectors, and the adapter may be operable to adapt the at least one signal from the test strip and apply an appropriate signal to the meter connectors and/or adapt the at least one signal from the test meter and apply an appropriate signal to the test strip.

Where there is the same number of meter interface connectors and test strip interface connectors, and the adapter may be operable to adapt the signal(s) from the test strip and apply an appropriate signal to the meter connectors.

Means may be provided for adapting one or more signals from the test strip for use by the meter.

Means may be provided for amplifying a signal from the test strip for applying to the meter.

The adapter may be operable to combine a plurality of signals from the test strip to create a reduced number of signals for applying to the test meter. The adapter may be operable to combine a plurality of signals from the meter to create a reduced number of signals for applying to the test strip. The adapter may be operable to adapt a plurality of signals from the test strip to create an increased number of signals for applying to the test meter. The adapter may be operable to adapt a plurality of signals from the meter to create an increased number of signals for applying to the test strip.

A power supply may be provided for supplying active components. Additionally or alternatively, a processor may be provided for processing one or more signals from the test strip for applying to the meter.

Means may be provided for physically securing the adapter to the meter and the test strip.

According to another aspect of the invention, there is provided a test strip for measuring the amount of analyte in a test sample, typically a biological fluid, including an integrally formed adapter for adapting an output from the test strip for use by a test meter; one or more connectors for connecting the adapter to test signal generating means on the strip and a meter interface for connecting the adapter and the meter.

The meter interface may have one or more connectors. The meter connectors and the test strip connectors may have different configurations, the adapter including means for connecting the meter interface connectors and the test strip interface connectors.

The connectors may be electrical connectors for passing electrical signals between the test strip and the adapter.

Where there are different numbers of meter interface connectors and test strip connectors, and the adapter may be operable to adapt the at least one signal from the test strip and apply an appropriate signal to the meter connectors.

Means may be provided for adapting one or more signals from the signal generating components of the test strip for use by the meter. Means may be provided for amplifying a signal from the tester for applying to the meter.

The adapter may be operable to combine a plurality of signals from the test signal generating means to create a reduced number of signals for applying to the test meter. Equally, the adapter may be operable to combine a plurality of signals from the meter to create a reduced number of signals for applying to the test signal generating means.

The adapter may be operable to adapt a plurality of signals from the test signal generating means to create an increased number of signals for applying to the test meter. The adapter may be operable to adapt a plurality of signals from the meter to create an increased number of signals for applying to the test signal generating means.

Power supply means may be provided for supplying active components. Means may be provided for physically securing the adapter to the meter.

According to another aspect of the invention, there is provided a method for testing a sample, for example a biological fluid, using a meter and a test strip that are Incompatible, the method comprising connecting the meter and the test strip using an adapter that Is operable to provide a compatibility bridge between the meter and the test strip.

### Brief Description of the Drawings

Various aspects of the invention will now be described by way of example only with reference to the accompanying drawings, of which:
Figure 1 is a plan view of a system for measuring analyte in a sample that includes a test strip and separate adapter and
Figure 2 is a plan view of a test strip with an Integrated adapter for use in measuring analyte in a sample.

### Specific Description of the Drawings

The present invention relates to an adaptor that is designed to provide an interface between an electronic readout meter and a test strip for the determination of the presence and or concentration of substance of interest in a fluid sample, for example glucose levels In a blood sample. The adapter is adapted to create compatibility between test strips and meters that would normally be incompatible. For example, the adapter can be configured to match different electrode configurations, electrode numbers, electrode sizes, test strip widths *l* thicknesses, among other things. The adaptor can be designed so that an alternative and lower cost test strip can be made compatible with an existing electronic meter originally designed for use with a more expansive test strip. By doing so, a test strip manufacturer can provide a lower cost test strip for use In an installed base of meters where test strip costs is of importance.

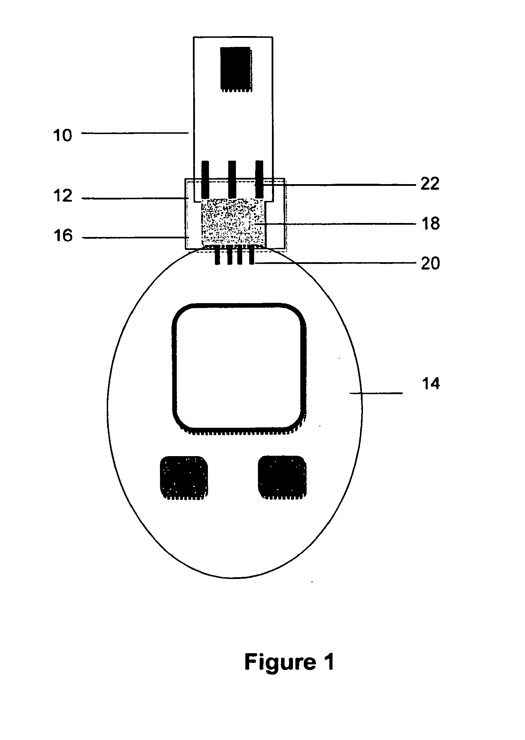

Figure 1 shows an example of a test strip 10 that is fitted with an adapter 12 for insertion into a meter 14 that is designed to operate in conjunction with a different test strip. The adapter 12 has an outer housing 16 that encloses electronic control and/or conversion circuitry 18. Extending from one end of the housing 16 is an electrical contact array 20 designed to fit into a strip port connector of the target meter 14. The array 20 is configured such that it closely replicates the array of contacts found on the end of the test strip 10 that is adapted for insertion Into the meter 14 for which it was originally designed. By inserting this set of protruding electrical contacts 20 into the meter 14 the adaptor 12 is either permanently or releasably attached to the target meter. This can be achieved using any suitable fixing.

At the other end of the adaptor housing 16 Is a strip port connector 22, which is designed to receive the insertible end of the test strip 10 to be used with the target meter 14. The strip port connector 22 has contacts (not shown) that are designed to match those disposed on the insertible end of the strip 10 to allow effective electrical and physical contact between the test strip and the adaptor 12, By inserting the strip into the adaptor's strip port connector the test strip is held In place.

Contained within the adaptor 12 is the electronic circuitry 18 that links the contacts of the alternative test strip 10 via the adaptor strip port connector 22 to the contacts disposed within the strip port connector of the target meter via the array of contacts 20 that is inserted into the strip port connector of the target meter 14. The electronic circuit 18 is such that upon application of a test sample on to the active area of the alternative test strip the signals produced by the alternative test strip 10 are converted in to a form expected by the target meter 14. For instance, the readings from the electrodes may be averaged, duplicated, amplified, corrected for certain errors or delayed as required.

By using an adaptor 12 as described above, it is possible to provide improved product performance to users without the need to replace a user's meter 14. For instance, in one embodiment an adaptor 12 may be designed to allow the signal produced by the test strip to be amplified so that the electrode sizes on the test strip can be substantially reduced. By reducing the electrode sizes, it is possible to reduce the blood volume requirement of the test strip thus reducing the pain associated with blood glucose testing. This could be done without requiring distribution of a new meter to users.

The accuracy of a test strip 10 may be improved by allowing a strip configured to remove the effects of interfering substances to be used with a meter not configured to provide this function. In this example, a test strip 10 can be designed that incorporates a background correction electrode in addition to the normal working electrode. An adaptor 12 can then be designed that to allow a reading from the background correction electrode to be subtracted from the reading provided by the normal working electrode before the working electrode signal is passed to the meter 14 via the adaptor 12. In this way, the background correction is transparent to the meter 14, thus allowing improved accuracy whilst still using the installed base of meters. Background correction allows the effects of interfering substances to be measured and removed from the glucose reading thus removing the system's sensitivity to commonly interfering substances such as ascorbic acid, paracetamol, gentisic acid, uric acid etc.

The adapter provides an interface between an electrochemical strip and an existing instrument and so adds functionality to the instrument. As well as re-configuring signals from the strip to the instrument, the adapter can be arranged to adapt signals from the instrument to the strip. For example, where the number of channels in the host instrument differs from that in the electrochemical strip, the adapter routes the signals available so that both instrument and electrochemical strip are satisfied, regardless of the direction of flow of the signals. In this case, the adapter may simply be arranged to reconfigure the electrical connections, for example providing connectors that merge, thereby to provide n connections at one end and n-x at the other.

In the case of an instrument that expects to measure electrical current, the adapter may be arranged to map the electrochemical strip's current generation characteristic to the range of current expected by the host instrument. Equally, the adapter could translate a voltage(s) output by the host instrument to a voltage or voltages expected by the electrochemical strip. Additionally or alternatively, the adapter module can translate timing requirements so that the electrochemical strip becomes compatible with the instrument's timing needs.

Where an activation is generated, for example when a strip is inserted into a meter, the adapter may operable to pass the activation signal from the meter or to the electrochemical strip via circuitry that provides electrical compatibility and could also be time shifted to allow for differing timing requirements between the two devices.

Other examples of ways in which the adapter could implement a strip/meter re-configuration or adaptation include current splitting or merging functions. For example, operational amplifiers in a current splitting configuration may be used to split a current source into a number of current sinks with a defined distribution in the direction of instrument to electrochemical strip or from electrochemical strip to instrument. In another example, a current merging configuration may be implemented to feed a current sink from a number of current sources with a defined distribution in the direction of instrument to electrochemical strip or from electrochemical strip to instrument. Any of the above could be achieved by using a microcontroller and Analog to Digital converters and Digital to Analog converters together with signal conditioning circuitry and controlled by a software program.

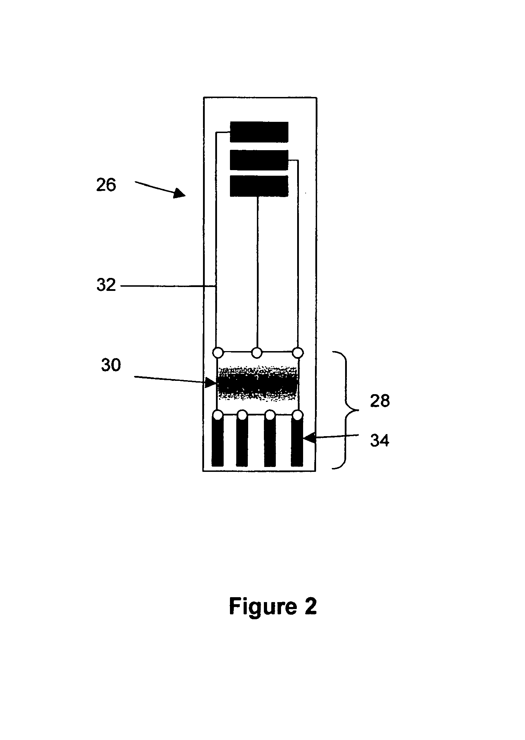

Although the adapter described with reference to Figure 1 is separate, but connectable to the test strip 10, it could also be integral with the strip 26. For example, the adapter 28 could be printed or laminated onto the end of the test strip 26 that is to be inserted into the meter. Figure 2 shows an example of this. In this case, the adapter 28 has electronics 30 that are printed, laminated or otherwise attached onto the end of the test strip 26. Connections 32 are formed between the adapter electronics 28 and the test strip electrodes along the length of the strip 26. Target meter specific contacts 34 are at the end of the strip that has to be inserted into the meter 14, As before, these are configured to match the target meter 14.

An advantage of having the adapter integrated with the test strip is that the electronic can be changed easily with no requirement for the user to change the adaptor. Also, the new strip change over is essentially transparent to the end user since there is no requirement for a discrete adaptor to be plugged into the meter. In addition, other functionality may be added to the strip such as calibration codes and expiry date information.

In the integrated test strip and adapter arrangement of Figure 2, the base test strip could be produced with a single design with a blank space at the lower end being left free for the addition of the specific electronic adaptor circuit and contacts. In this way, one design of test strip can be "flavoured" to fit in to a number of different target meters.

Various modifications and variations to the described embodiments of the invention will be apparent to those skilled in the art. For example, the adapter module could be powered by the host instrument or by its own power supply such as a battery (not shown). Although the invention has been described in connection with specific preferred embodiments, it should be understood that the invention as claimed should not be unduly limited to such specific embodiments. Indeed, various modifications of the described modes of carrying out the invention obvious to those skilled in the art are covered by the present invention.

## Claims

1. An adapter (12) for an analyte measuring device for measuring the amount of analyte in a test sample, typically a biological fluid, comprising a meter (14) and a test strip (10), the adapter (12) comprising a meter interface (20) for connecting to a meter (14) designed for use with a test strip of one design, and a test strip interface (22) for connecting to a test strip (10) of a different design, and means for adapting the at least one output from the test strip (10) of different design for use by the test meter (14) and/or an at least one output from the test meter (14) for use by the test strip (10) of different design.

2. An adapter (12) as claimed in claim 1 wherein the meter interface (20) and the test strip interface (22) comprise one or more connectors, the meter interface connectors and the test strip interface connectors having different configurations, the adapter including means for connecting the meter interface connectors and the test strip interface connectors.

3. An adapter (12) as claimed in claim 2 wherein the connectors are electrical connectors for passing electrical signals between the test strip (10) and the meter (14).

4. An adapter (12) as claimed in claim 1 wherein the meter interface (20) and the test strip interface (22) comprise one or more connectors, the meter interface connectors and the test strip interface connectors having the substantially the same configurations, the adapter (12) including means for connecting the meter interface connectors and the test strip interface connectors.

5. An adapter (12) as claimed in claim 4, wherein the connectors are electrical connectors for passing electrical signals between the test strip (10) and the meter (14).

6. An adapter (12) as claimed in any of claims 1 to 3 wherein there are different numbers of meter interface connectors and test strip interface connectors, and the adapter (12) is operable to adapt the at least one signal from the test strip (10) and apply an appropriate signal to the meter connectors and/or adapt the at least one signal from the test meter (14) and apply an appropriate signal to the test strip (10).

7. An adapter (12) as claimed in any of claims 1 to 5 wherein the number of meter interface connectors and test strip interface connectors is the same, and the adapter (12) is operable to adapt the at least one signal from the test strip (10) and apply an appropriate signal to the meter connectors.

8. An adapter (12) as claimed in any of the preceding claims comprising means for adapting one or more signals from the test strip (10) for use by the meter (14).

9. An adapter (12) as claimed in claim 8 further comprising means for amplifying a signal from the test strip (10) for applying to the meter (14).

10. An adapter (12) as claimed in any of the preceding claims operable to combine a plurality of signals from the test strip (10) to create a reduced number of signals for applying to the test meter (14) and/or operable to combine a plurality of signals from the meter (14) to create a reduced number of signals for applying to the test strip (10).

11. An adapter (12) as claimed in any of the preceding claims operable to adapt a plurality of signals from the test strip (10) to create an increased number of signals for applying to the test meter (14) and/or operable to adapt a plurality of signals from the meter (14) to create an increased number of signals for applying to the test strip (10).

12. An adapter (12) as claimed in any of the preceding claims comprising a power supply for supplying active components and/or a processor for processing one or more signals from the test strip (10) for applying to the meter (14) and/or means for physically securing the adapter (12) to the meter (14) and the test strip (10).

13. A test strip (10) for measuring the amount of analyte in a test sample, typically a biological fluid, including an adapter (12) as claimed in any of the preceding claims, wherein the adapter (12) is integrally formed onto the test strip (10).

14. An adapter (12) or test strip (10) of any of the preceding claims wherein the analyte is glucose.

15. A method for testing a sample, for example a biological fluid, using a meter (14) and a test strip (10) that are incompatible, the method comprising connecting the meter (14) and the test strip (10) using an adapter (12) that is operable to provide a compatibility bridge between the meter (14) and the test strip (10).

## Patentansprüche

1. Adapter (12) für eine Analytmessvorrichtung für das Messen der Menge des Analyts in einer Probe, typischerweise einem biologischen Fluid, die ein Messgerät (14) und einen Probestreifen (10) aufweist, wobei der Adapter (12) aufweist: einen Messgeräteanschluss (20) für das Verbinden mit einem Messgerät (14), ausgelegt für eine Verwendung mit einem Probestreifen einer Ausführung; und einen Probestreifenanschluss (22) für das Verbinden mit einem Probestreifen (10) einer anderen Ausführung; und eine Einrichtung für das Anpassen des mindestens einen Ausgangs vom Probestreifen (10) einer anderen Ausführung für eine Verwendung durch das Messgerät (14) und/oder mindestens einen Ausgang vom Messgerät (14) für eine Verwendung durch den Probestreifen (10) der anderen Ausführung.

2. Adapter (12) nach Anspruch 1, bei dem der Messgeräteanschluss (20) und der Probestreifenanschluss (22) einen oder mehrere Verbinder aufweisen, wobei die Verbinder des Messgeräteanschlusses und die Verbinder des Probestreifenanschlusses unterschiedliche Konfigurationen aufweisen, wobei der Adapter eine Einrichtung für das Verbinden der Verbinder des Messgeräteanschlusses und der Verbinder des Probestreifenanschlusses umfasst.

3. Adapter (12) nach Anspruch 2, bei dem die Verbinder elektrische Verbinder für das Führen elektrischer Signale zwischen dem Probestreifen (10) und dem Messgerät (14) sind.

4. Adapter (12) nach Anspruch 1, bei dem der Messgeräteanschluss (20) und der Probestreifenanschluss (22) einen oder mehrere Verbinder aufweisen, wobei die Verbinder des Messgeräteanschlusses und die Verbinder des Probestreifenanschlusses im Wesentlichen die gleichen Konfigurationen aufweisen, wobei der Adapter (12) eine Einrichtung für das Verbinden der Verbinder des Messgeräteanschlusses und der Verbinder des Probestreifenanschlusses umfasst.

5. Adapter (12) nach Anspruch 4, bei dem die Verbinder elektrische Verbinder für das Führen elektrischer Signale zwischen dem Probestreifen (10) und dem Messgerät (14) sind.

6. Adapter (12) nach einem der Ansprüche 1 bis 3, bei dem eine unterschiedliche Anzahl von Verbindern des Messgeräteanschlusses und Verbindern des Probestreifenanschlusses vorhanden ist, und wobei der Adapter (12) funktionsfähig ist, um das mindestens eine Signal vom Probestreifen (10) anzupassen und ein geeignetes Signal an den Messgeräteverbindern anzulegen, und/oder um das mindestens eine Signal vom Messgerät (14) anzupassen und ein geeignetes Signal am Probestreifen (10) anzulegen.

7. Adapter (12) nach einem der Ansprüche 1 bis 5, bei dem die Anzahl der Verbinder des Messgeräteanschlusses und der Verbinder des Probestreifenanschlusses die gleiche ist, und wobei der Adapter (12) funktionsfähig ist, um das mindestens eine Signal vom Probestreifen (10) anzupassen und ein geeignetes Signal an den Messgeräteverbindern anzulegen.

8. Adapter (12) nach einem der vorhergehenden Ansprüche, der ein Mittel für das Anpassen von einem oder mehreren Signalen vom Probestreifen (10) für eine Benutzung durch das Messgerät (14) aufweist.

9. Adapter (12) nach Anspruch 8, der außerdem ein Mittel für das Verstärken eines Signals vom Probestreifen (10) für ein Anlegen am Messgerät (14) aufweist.

10. Adapter (12) nach einem der vorhergehenden Ansprüche, der funktionsfähig ist, um eine Vielzahl von Signalen vom Probestreifen (10) zu kombinieren, um eine verringerte Anzahl von Signalen für ein Anlegen am Messgerät (14) zu erzeugen, und/oder funktionsfähig ist, um eine Vielzahl von Signalen vom Messgerät (14) zu kombinieren, um eine verringerte Anzahl von Signalen für ein Anlegen am Probestreifen (10) zu erzeugen.

11. Adapter (12) nach einem der vorhergehenden Ansprüche, der funktionsfähig ist, um eine Vielzahl von Signalen vom Probestreifen (10) anzupassen, um eine erhöhte Anzahl von Signalen für ein Anlegen am Messgerät (14) zu erzeugen, und/oder funktionsfähig ist, um eine Vielzahl von Signalen vom Messgerät (14) anzupassen, um eine erhöhte Anzahl von Signalen für ein Anlegen am Probestreifen (10) zu erzeugen.

12. Adapter (12) nach einem der vorhergehenden Ansprüche, der aufweist: eine Stromversorgung für das Versorgen der aktiven Bauteile und/oder eines Prozessors für das Verarbeiten von einem oder mehreren Signalen vom Probestreifen (10) für ein Anlegen am Messgerät (14); und/oder ein Mittel für das physikalische Sichern des Adapters (12) am Messgerät (14) und dem Probestreifen (10).

13. Probestreifen (10) für das Messen der Menge des Analyts in einer Probe, typischerweise einem biologischen Fluid, der einen Adapter (12) nach einem der vorhergehenden Ansprüche umfasst, wobei der Adapter (12) zusammenhängend am Probestreifen (10) ausgebildet ist.

14. Adapter (12) oder Probestreifen (10) nach einem der vorhergehenden Ansprüche, wobei der Analyt Glucose ist.

15. Verfahren zur Prüfung einer Probe, beispielsweise eines biologischen Fluids, bei Anwendung eines Messgerätes (14) und eines Probestreifens (10), die inkompatibel sind, wobei das Verfahren den Schritt des Verbindens des Messgerätes (14) und des Probestreifens (10) bei Verwendung eines Adapters (12) aufweist, der funktionsfähig ist, um eine Kompatibilitätsbrücke zwischen dem Messgerät (14) und dem Probestreifen (10) bereitzustellen.

## Revendications

1. Adaptateur (12) pour un dispositif de mesure d'un analyte, pour mesurer la quantité de l'analyte dans un échantillon de test, typiquement un fluide biologique, comprenant un dispositif de mesure (14) et une bandelette de test (10), l'adaptateur (12) comprenant une interface du dispositif de mesure (20), en vue de la connexion à un dispositif de mesure (14) configuré de sorte à être utilisé avec une bandelette de test d'une conception, et une interface de la bandelette de test (22) en vue de la connexion à une bandelette de test (10) de conception différente, et un moyen pour adapter la au moins une sortie de la bandelette de test (10) de conception différente, en vue de l'utilisation par le dispositif de mesure de test (14), et/ou la au moins une sortie du dispositif de mesure de test (14) en vue de l'utilisation par la bandelette de test (10) de conception différente.

2. Adaptateur (12) selon la revendication 1, dans lequel l'interface du dispositif de mesure (20) et l'interface de la bandelette de test (22) comprennent un ou plusieurs connecteurs, les connecteurs de l'interface du dispositif de mesure et les connecteurs de l'interface de la bandelette de test ayant des configurations différentes, l'adaptateur englobant un moyen pour connecter les connecteurs de l'interface du dispositif de mesure et les connecteurs de l'interface de la bandelette de test.

3. Adaptateur (12) selon la revendication 2, dans lequel les connecteurs sont des connecteurs électriques destinés à faire passer des signaux électriques entre la bandelette de test (10) et le dispositif de mesure (14).

4. Adaptateur (12) selon la revendication 1, dans lequel l'interface du dispositif de mesure (20) et l'interface de la bandelette de test (22) comprennent un ou plusieurs connecteurs, les connecteurs de l'interface du dispositif de mesure et les connecteurs de l'interface de la bandelette de test ayant essentiellement les mêmes configurations, l'adaptateur (12) englobant un moyen pour connecter les connecteurs de l'interface du dispositif de mesure et les connecteurs de l'interface de la bandelette de test.

5. Adaptateur (12) selon la revendication 4, dans lequel les connecteurs sont des connecteurs électriques, destinés à faire passer des signaux électriques entre la bandelette de test (10) et le dispositif de mesure (14).

6. Adaptateur (12) selon l'une quelconque des revendications 1 à 3, comportant des nombres différents de connecteurs de l'interface du dispositif de mesure et de connecteurs de l'interface de la bandelette de test, l'adaptateur (12) servant à adapter le au moins un signal de la bandelette de test (10) et à appliquer un signal approprié aux connecteurs du dispositif de mesure, et/ou à adapter le au moins un signal du dispositif de mesure (14) et à appliquer un signal approprié à la bandelette de test (10).

7. Adaptateur (12) selon l'une quelconque des revendications 1 à 5, dans lequel le nombre de connecteurs de l'interface du dispositif de mesure et de connecteurs de l'interface de la bandelette de test est identique, l'adaptateur (12) servant à adapter le au moins un signal de la bandelette de test (10) et à appliquer un signal approprié aux connecteurs du dispositif de mesure.

8. Adaptateur (12) selon l'une quelconque des revendications précédentes, comprenant un moyen pour adapter un ou plusieurs signaux de la bandelette de test (10) en vue de leur utilisation par le dispositif de mesure (14).

9. Adaptateur (12) selon la revendication 8, comprenant en outre un moyen pour amplifier un signal de la bandelette de test (10) en vue de son application au dispositif de mesure (14).

10. Adaptateur (12) selon l'une quelconque des revendications précédentes, servant à combiner plusieurs signaux de la bandelette de test (10) pour créer un nombre réduit de signaux en vue de leur application au dispositif de mesure et test (14), et/ou servant à combiner plusieurs signaux du dispositif de mesure (14) pour créer un nombre réduit de signaux en vue de leur application à la bandelette de test (10).

11. Adaptateur (12) selon l'une quelconque des revendications précédentes, servant à adapter plusieurs signaux de la bandelette de test (10) pour créer un nombre accru de signaux en vue de leur application au dispositif de mesure de test (14), et/ou servant à adapter plusieurs signaux du dispositif de mesure (14) pour créer un nombre accru de signaux en vue de leur application à la bandelette de test (10).

12. Adaptateur (12) selon l'une quelconque des revendications précédentes, comprenant un bloc d'alimentation pour alimenter des composants actifs et/ou un processeur, pour assurer le traitement d'un ou de plusieurs signaux de la bandelette de test (10) en vue de leur application au dispositif de mesure (14), et/ou un moyen pour assurer la fixation physique de l'adaptateur (12) au dispositif de mesure (14) et à la bandelette de test (10).

13. Bandelette de test (10) pour mesurer la quantité d'un analyte dans un échantillon de test, typiquement un fluide biologique, englobant un adaptateur (12) selon l'une quelconque des revendications précédentes, l'adaptateur (12) étant formé d'une seule pièce sur la bandelette de test (10).

14. Adaptateur (12) ou bandelette de test (10) selon l'une quelconque des revendications précédentes, dans lequel l'analyte est du glucose.

15. Procédé d'analyse d'un échantillon, par exemple d'un fluide biologique, utilisant un dispositif de mesure (14) et une bandelette de test (10) qui sont incompatibles, le procédé comprenant l'étape de connexion du dispositif de mesure (14) et de la bandelette de test (10) par l'intermédiaire d'un adaptateur (12) servant à établir une liaison de compatibilité entre le dispositif de mesure (14) et la bandelette de test (10).
